# EUROPÄISCHE PATENTSCHRIFT

(11) **EP 2 012 835 B1**
(45) Veröffentlichungstag und Bekanntmachung des Hinweises auf die Patenterteilung: **09.02.2011**
(21) Anmeldenummer: 07727826.5
(22) Anmeldetag: 05.04.2007
(51) Int. Cl.: A61L 2/04

(54) **VERFAHREN ZUR STERILISATION UNTER VERWENDUNG VON SCHAUMSTOFFEN AUF BASIS EINES AMINOPLASTEN ALS STERILISIERBARES ARBEITSMITTEL**
METHOD FOR STERILISATION USING FOAMED MATERIALS BASED ON AMINOPLASTS AS STERILIZABLE WORKING MEANS
PROCÉDÉ DE STÉRILISATION UTILISANT DES MOUSSES À BASE D'AMINOPLASTE COMME OUTIL DE TRAVAIL POUVANT ÊTRE STÉRILISÉ

(30) Priorität: 18.04.2006 EP 06112698
(43) Veröffentlichungstag der Anmeldung: 14.01.2009
(73) Patentinhaber: BASF SE, 67056 Ludwigshafen (DE)
(72) Erfinder: QUADBECK-SEEGER, Hans-Jürgen, 67098 Bad Dürkheim (DE)
(86) Internationale Anmeldenummer: PCT/EP2007/053358
(87) Internationale Veröffentlichungsnummer: WO 2007/118803

(56) Entgegenhaltungen:
- EP-A- 0 733 404
- EP-A1- 0 633 283
- WO-A-01/94436
- DE-A1- 10 204 221
- GB-A- 775 296
- JP-A- 2000 288 482
- US-A1- 2005 136 238
- US-A1- 2005 202 232
- LABOR FÜR RADIOISOTOPEN,GEORG-AUGUST-UNIVERSITÄT GÖTTINGEN: "Einige Grundtechniken für die Kultur von Mikroorganismen"[Online] 12. Mai 2004 (2004-05-12), XP002438956 Gefunden im Internet: URL:http://www.radioisotope.de/pdf/LMP/tec hniken.pdf> [gefunden am 2007-06-22]

## Beschreibung

Die Erfindung betrifft Verfahren zur Sterilisation von Arbeitsgefäßen für medizinische oder mikrobiologische Arbeiten und zur Dekontamination von mit Mikroorganismen kontaminiertem Material bei Temperaturen über 100°C.

In der Mikrobiologie spielen steriles Arbeiten und die Sterilisation von Arbeitsmaterial eine wichtige Rolle. Für das Arbeiten werden Materialien, wie Wattebäusche zum Verschluss von Gläsern und Unterlagen durch Erhitzen in druckfesten Sterilisatoren bei 128°C und 4 Atm Druck sterilisiert und verpackt vermarktet.

Ebenso wichtig ist die Entsorgung von kontaminiertem Material. Insbesondere beim Arbeiten mit pathogenen Mikroorganismen und genetisch veränderten Mikroorganismen gelten strenge Vorschriften.

Bakterien und Pilze werden auf festen oder in flüssigen Nährmedien vermehrt. Diese müssen vor dem Beimpfen sterilisiert werden. Zur Abtötung von Mikroorganismen wird in der Regel bei hohen Temperaturen im Autoklaven behandelt. Sterile bzw. zu sterilisierende Behältnisse werden mit Watte- oder Zellstoffstopfen, Überfalldeckeln oder Schraubverschlüssen gegen Infektionen geschützt. Glas- und Metallgerät kann trocken in Metall- oder anderen temperaturbeständigen Behältern sterilisiert werden. Glaspipetten werden trocken in speziellen Dosen sterilisiert (Labor für Radioisotopen, Georg-August-Universität Göttingen, "Einig Grundtechniken für die Kultur von Mlikroorganismen")

Steril zu haltende Kulturröhrchen, Flaschen und Kolben werden mit Stopfen aus Watte gerolltem Zellstoff oder Silikonschaum verschlossen, die einerseits Luftzutritt ermöglichen, andererseits aber als Tiefenfilter Luftkeime zurückhalten. Sie dürfen allerdings keinerlei Feuchtigkeit enthalten, weil sonst Mikroorganismen von der Außenseite her nach innen durchwachsen können. Über kürzere Zeiten von etwa 2-3 Tagen wird Sterilität, wie bei Petrischalen, auch durch eng sitzende Überfallkappen aus z. b. Aluminium gewährleistet.

Aufgabe der Erfindung war es Verfahren unter Verwendung einfach sterilisierbare 8 Arbeitsmittel für die Mikrobiologie bereitzustellen. Das Arbeitsmittel sollte insbesondere als Verschluss für mikrobiologische Arbeitsgefäße geeignet und bei hohen Temperaturen auch ohne Druck sterilisierbar sein.

Demgemäss wurden die Verfahren gemäss den Ansprüchen 1 und 2 gefunden.

Als offenzellige Schaumstoffe werden bevorzugt elastische Schaumstoffe auf Basis eines Melamin/Formaldehyd-Kondensationsproduktes mit einer spezifischen Dichte von 5 bis 100 g/l, insbesondere von 8 bis 20 g/l verwendet. Die Zellzahl liegt üblicherweise im Bereich von 50 bis 300 Zellen/25 mm. Die Zugfestigkeit liegt bevorzugt im Bereich von 100 bis 150 kPa und die Bruchdehnung im Bereich von 8 bis 20%.

Zur Herstellung kann nach EP-A 071 672 oder EP-A 037 470 eine hochkonzentrierte, treibmittelhaltige Lösung oder Dispersion eines Melamin-Formaldehyd-Vorkondensates mit Heißluft, Wasserdampf oder durch Mikrowellenbestrahlung verschäumt und ausgehärtet werden. Derartige Schaumstoffe sind im Handel unter der Bezeichnung Basotect® der Firma BASF Aktiengesellschaft erhältlich.

Das Molverhältnis Melamin/Formaldehyd liegt im allgemeinen im Bereich von 1 : 1 bis 1:5. Zur Herstellung besonders formaldehydarmer Schaumstoffe wird das Molverhältnis im Bereich von 1 : 1,3 bis 1 : 1,8 gewählt und ein sulfitgruppenfreies Vorkondensat eingesetzt, wie z. B in WO 01/94436 beschrieben.

Um die anwendungstechnischen Eigenschaften zu verbessern, können die Schaumstoffe anschließend getempert und verpresst werden. Die Schaumstoffe können zur gewünschten Form und Dicke zugeschnitten und ein- oder beidseitig mit Deckschichten kaschiert werden. Beispielsweise kann eine Polymer- oder Metallfolie als Deckschicht aufgebracht werden.

Der offenzellige Schaumstoff kann aufgrund der weitgehenden chemischen Beständigkeit des Melamin/Formaldehyd-Kondensats auch direkt mit verschiedenen Chemikalien oder kryogenen Flüssigkeiten in Kontakt kommen.

Der Schaumstoff kann hydrophob ausgerüstet sein. Es kann der Zusatz von Hydrophobierungsmitteln während der Schaumbildung oder durch eine Nachimprägnierung mit einem Hydrophobierungsmittel erfolgen. Geeignete Hydrophobierungsmittel sind beispielsweise Silikone, Paraffine. Fluorcarbonharze, oder Fluor- oder Silikontenside. Da mikrobiologische Arbeiten zu einem großen Teil in wässrigem Milieu durchgeführt werden, ist eine hydrophobe Ausrüstung des Schaumstoffs, wie in EP-A 633 283 beschrieben, zur Senkung der Wasseraufnahme in vielen Fällen vorteilhaft.

Der Schaumstoff kann mit Garnen und daraus hergestellte Geweben aus 5 bis 90 Gew.-% Melaminfasern, 5 bis 90 Gew.-% Naturfasern und 0,1 bis 30 Gew.-% Polyamidfasern kombiniert werden, wie sie in WO 02/10492 beschrieben werden. Aufgrund der hohen thermischen, chemischen und mechanischen Stabilität dieser Fasern kann beispielsweise die Festigkeit des Schaumstoffs erhöht werden, ohne die Sterilisierbarkeit negativ zu beeinträchtigen.

Aufgrund der Elastizität des offenzelligen Schaumstoffes kann dieser auf einfache Weise in bereits vorgefertigte Behälterteile eingefügt werden. Selbst bei tiefen Temperaturen, beispielsweise unter -80°C bleibt der Schaumstoff elastisch. Eine Schädigung durch Verspröden tritt nicht auf.

Erfindungsgemäß kann mit dem offenzelligen Schaumstoff auf Basis eines Aminoplasten die Öffnung eines Arbeitsgefäßes, beispielsweise ein Kulturröhrchen, Flasche oder Kolben für medizinische oder mikrobiologische Arbeiten, verschlossen werden und zur Sterilisation bei Temperaturen über 100°C behandelt werden.

Es ist auch möglich mit Mikroorganismen kontaminiertem Material in ein Arbeitsgefäß einzubringen, mit dem offenzelligen Schaumstoff auf Basis eines Aminoplasten zu verschließen und durch Behandlung bei Temperaturen über 100°C zu dekontaminieren.

Die Behandlung kann im Autoklaven unter Wasserdampf bei 120 bis 140°C und Druck im Bereich von 1 bis 4 bar durchgeführt werden. Aus dem Autoklavenraum muss die Luft vollständig durch Wasserdampf verdrängt werden. Entscheidend für den Sterilisationserfolg ist die Höhe der Temperatur, nicht der Überdruck, der nur erforderlich ist, um Temperaturen von über 100 °C zu erreichen. 134°C können nur bei entsprechend unempfindlichen Materialien angewendet werden.

Für die Sterilisation kleiner Flüssigkeitsvolumina unter 20 ml sind bei 121°C in der Regel mindestens 15 Minuten reine Sterilisationszeit, ohne die im Sterilisationsgut erforderliche Aufheizzeit, erforderlich. Bei Einzelvolumina von etwa 50 ml bis 1 l müssen Aufheizzeiten von 5 bis 40 Minuten zugeschlagen werden. Bei modernen Autoklaven wird die eingestellte Sterilisationszeit über Temperaturfühler im Sterilisationsgut kontrolliert.

Zu sterilisierende Gefäße dürfen nicht dicht verschlossen sein, damit beim Aufheizen Luft und Wasserdampf entweichen und beim Abkühlen einströmen können. Sonst leere Gefäße sollten etwas Wasser enthalten, damit sie sich mit Wasserdampf füllen. Als vorteilhaft erweist sich hierbei der erfindungsgemäß verwendete offenzellige Schaumstoff, da dieser nicht wie Watte- und Zellstoffstopfen mit Pergamentpapier oder Alufolie vor herabtropfendem Kondenswasser geschützt werden muss. Alle Verschlüsse, auch Schraubverschlüsse, dürfen auch von innen nicht feucht werden, was z.B. durch Hochkochen von Flüssigkeit geschehen kann. Deswegen dürfen Gefäße allerhöchstens zu ¾ gefüllt sein. Nach Ablauf der Sterilisationszeit muss der Autoklav ungeöffnet auf unter 100°C abgekühlt werden, damit Flüssigkeiten bei Druckentlastung nicht zu sieden beginnen. Autoklaven müssen heute gegen das Öffnen bei Temperaturen über 80°C gesichert sein.

Bevorzugt wird die Behandlung jedoch trocken, d. h. ohne Wasserdampf bei einer Temperatur im Bereich von 140 bis 220°C durchgeführt. Aufwand und Arbeitsabläufe können dadurch erheblich reduziert werden. Glas- und Metallgerät kann, verpackt in Pergamentpapier oder Alufolie, trocken sterilisiert werden. Dafür sind üblicherweise bei 150°C um 3 Stunden, bei 180°C um 30 Minuten ausreichend. Die hinzuzurechnenden Aufheizzeiten richten sich nach der Größe der Einzelteile und nach dem Füllungsgrad des Sterilisatorraumes. Bei großen aufzuheizenden Massen sind dies mehrere Stunden. Am zweckmäßigsten ist die Sterilisation nachmittags zu beginnen, so dass der Sterilisator über Nacht langsam abkühlen kann. Glaspipetten werden in speziellen zweiteiligen Aluminium- oder Edelstahldosen sterilisiert.

Sterilisatoren unterscheiden sich von reinen Trockenschränken durch das Vorhandensein eines Umluftventilators, der vor allem bei dichter Packung des Sterilisationsgutes eingeschaltet sein sollte.

Der erfindungsgemäß verwendete Schaumstoff zeichnet sich durch eine hohe Temperaturstabilität aus, absorbiert aber keine Mikrowellenstrahlung. Er ist somit auch zur Sterilisation durch Einbringen von Mikrowellenenergie geeignet.

Nach dem Öffnen und vor dem Wiederverschließen steriler oder Reinkulturen enthaltender Gefäße flammt man Öffnung und Stopfen zur Sterilisation kurz mit dem Bunsenbrenner ab. Im Gegensatz zu dem erfindungsgemäß verwendeten offenzelligen Schaumstoff können locker gestopfte Wattestopfen hierbei Feuer fangen.

Der erfindungsgemäße verwendete offenzellige Schaumstoff zeichnet sich durch eine hohe Thermostabilität bei Temperaturen bis 180°C aus. Er ist leicht in geläufigen Sterilisatoren zu sterilisieren. Kurzfristig kann er sogar auf 220°C erhitzt werden. Da Mikroorganismen in der Regel eine Erhitzung auf 150°C nicht überleben, kann der erfindungsgemäß verwendete offenzellige Schaumstoff auch drucklos in einem Thermoschrank oder einem Backofen, die es als Tischmodelle gibt, die notwendige Zeit auf 150° bis 200°C erhitzt werden. Aufwand und Arbeitsabläufe können dadurch erheblich reduziert werden. Der Schaumstoff ist stark komprimierbar und elastisch, daher können auch grobe Zuschnitte passgenau in Öffnungen variabler Durchmesser eingesetzt werden.

### Beispiele:

Folgende Materialien wurden als Stopfen in der Kultur von Mikroorganismen eingesetzt:

### Beispiel 1:

Zugeschnittene Stopfen eines offenzelligen Melamin/Formaldehyd-Schaumstoff mit einer Dichte von etwa 10 kg/m³ (Basotect® der BASF Aktiengesellschaft)

### Vergleichsversuch V1:

Konventionelle Wattestopfen (27-32,5 mm; Buddeberg, # 1012700)

### Vergleichsversuch V2:

Kunststoffstopfen mit permeabler Membran (Buddeberg)

### 1. Abflämmbarkeit

Wattestopfen (V1) sind für eine Sterilisation mittels Flamme völlig ungeeignet und beginnen sofort zu brennen. Kunststoff (V2)- und Basotect® -Stopfen (Beispiel 1) können dagegen bei Bedarf entsprechend vorbehandelt werden.

### 2. Autoklavierbarkeit/Sterilität

Der erfindungsgemäße Stopfen (Beispiel 1) und die Vergleichsstopfen (V1, V2) wurden unter Standardbedingungen autoklaviert (121°C/20 min; trockene oder feuchte Hitze) und auf Erlenmeyerkolben, gefüllt mit LB-Wachstumsmedium, ohne Zusatz von Mikroorganismen bei 37°C, 150 rpm für 7 Tage inkubiert. Bei keinem Ansatz konnte eine Veränderung des Stopfens durch die Hitzebehandlung bzw. eine Kontamination im Medium während der Inkubation festgestellt werden.

### 3. Sauerstoffdurchlässigkeit

Die Sauerstoffdurchlässigkeit der 3 verschiedenen Stopfen wurde mittels eines Sulfitdetektionssystems detektiert. Hierzu wurde in einem 1 L Erlenmeyerkolben 100 ml Sulfitlösung zugeben und bei RT/150 rpm inkubiert. Der Zeitpunkt des Farbumschlages ist ein Maß für die Sauerstoffdurchlässigkeit.

### Zubereitung des 0,5 M Sulfitsystems

Herstellung. Das Standard 0,5 M Sulfitsystem (Natriumsulfit ≧98 %, Best.-Nr. 60860, Roth, Karlsruhe) wird nach Hermann et al, (Biotech Bioeng,.2001) mit 0,012 M Phosphatpuffer; 10⁻⁷ M Kobaltsulfat; 0,015 g/L Bromthymolblaulösung (Best.-Nr. 18460, Fluka, BuchslCH) in mit Stickstoff (5-10 min) entgasten VE Wasser angesetzt.

Zur Herstellung von 1000 mL 0,5 M Sulfitlösung werden 24 mL 0,5 M Phosphatpufferlösung (siehe unten) auf 1000 mL aufgefüllt. Von dieser 0,012 M Phosphatpufferlösung werden 800 mL mit Stickstoff begast. 63 g Natriumsulfit werden in der 800 mL Lösung aufgelöst und 15 mL Bromthymolblaulösung mit einer Konzentration von 1 g/L zugegeben (siehe unten). 2 mL 5.10⁻⁵ M Kobaltlösung werden hinzugegeben und mit dem Rest der 0,012 M Phosphatpufferlösung auf 1000 mL aufgefüllt. Der pH-Wert wird mit Schwefelsäure (30%, Best.-Nr. A2712.500, Applichem, Darmstadt) auf pH 8 eingestellt. Während der Zubereitung wird die Lösung stets mit Stickstoff begast.

### i) Ansatz der 0,5 M Phosphatpufferlösung

### Ansatz von 500 mL 0,5 M Na₂HPO₄-Lösung

35,490 g Na₂HPO₄- (Dinatriumhydrogenphosphat wasserfrei ≧99%, Best.-Nr. P030.1, Roth, Karlsruhe) werden in 450 mL VE-Wasser gelöst und auf 500 mL aufgefüllt.

### Ansatz von 50 mL 0,5 M NaH₂PO₄-Lösung

3,450 g NaH₂PO₄ (Natriumdihydrogenphosphat Monohydrat ≧98 %, Best.-Nr. K300.2, Roth, Karlsruhe) werden in 45 mL VE-Wasser gelöst und auf 50 mL aufgefüllt.

Die angesetzten 500 mL 0,5 M Na₂HPO₄-Lösung werden durch Zugabe der 0,5 M NaH₂PO₄-Lösung (ca. 40- 50 mL) bis auf pH = 8 eingestellt.

### ii) Ansatz des Kobaltkatalysators

5.10⁻³ mol/L (Stammlösung): 140,55 mg CoSO₄ *7 H20 (≧97,5%, Best.-Nr. 60860, Fluka Chemie AG, Buchs/CH) werden mit VE Wasser auf 100 mL aufgefüllt.

5.10⁻³ mol/L (Endlösung): 1 mL aus obiger Stammlösung werden mit VE Wasser auf 100 mL aufgefüllt.

### iii) Ansatz der Bromthymolblaulösung

0,1 g Bromthymolblau (Best. Nr. 18460, Fluka, Buchs/CH) wird auf 100 mL Wasser aufgefüllt und ca. 1 h gerührt (=1 g/L).

Kunststoffstopfen (V2) und Basotect® -Stopfen (Bsp. 1) zeigen ähnliches Verhalten (Farbumschlag nach 11-13 Stunden), während Wattestopfen (V1) mit 17-20 Stunden signifikant schlechter sind. Limitierungen im Sauerstofftransfer können zu ungenügendem Wachstum oder Veränderungen im Metabolismus der Mikroorganismen führen und sind unerwünscht. Basotect®- bzw. Kunststoffstopfen sind also deutlich besser geeignet.

### 4. Verdunstungsverluste

Es wurden mehrere Ansätze mit 1 L Erlenmeyerkolben, gefüllt mit jeweils 150 ml LB-Medium, bei 37°C/150 rpm inkubiert. Die Verdunstungsverluste nach 7 Tagen lage bei den Watte (V1)- und Kunststoffstopfen (V2) bei ca. 10-11 ml, während durch das grobporige Basotect® ca. 12-14 ml verloren gehen. Die minimal erhöhte Verdunstung wird durch die anderen positiven Eigenschaften von Basotect® überkompensiert und ist der Größenordnung nach nicht kritisch.

### 5. Handhabung

Wattestopfen(V1) und auch Kunststoffstopfen(V2) sind sehr starr und es besteht die Gefahr, dass sie wieder von den Kolben abfallen (besonders beim Schütteln mit hoher Drehzahl). Bei Nutzung dieser Stopfen mit hohem Kraftaufwand liegt weiterhin eine potenzielle Verletzungsgefahr vor. Basotect®-Stopfen lassen sich mit minimalem Kraftaufwand leicht auf die Kolbenöffnungen aufbringen und sind optimal angepasst.

## Patentansprüche

1. Verfahren zur Sterilisation von Arbeitsgefäßen, **dadurch gekennzeichnet, dass** die Öffnung des Arbeitsgefäßes mit einem offenzelligen Schaumstoff auf Basis eines Aminoplasten verschlossen und bei Temperaturen über 100°C behandelt wird.

2. Verfahren zur Dekontamination von mit Mikroorganismen kontaminiertem Material durch Behandlung bei Temperaturen über 100°C, **dadurch gekennzeichnet, dass** man das Material vor der Behandlung in ein Arbeitsgefäß einbringt und mit einem offenzelligen Schaumstoff auf Basis eines Aminoplasten verschließt.

3. Verfahren nach Anspruch 1 oder 2, **dadurch gekennzeichnet, dass** die Öffnung des Arbeitsgefäßes mit einem offenzelligen Schaumstoff auf Basis eines Melamin/Formaldehyd-Kondensationsproduktes verschlossen wird.

4. Verfahren nach einem der Ansprüche 1 bis 3, **dadurch gekennzeichnet, dass** der offenzellige Schaumstoff eine spezifische Dichte im Bereich von 5 bis 100 g/l aufweist.

5. Verfahren nach einem der Ansprüche 1 bis 4, **dadurch gekennzeichnet, dass** der offenzellige Schaumstoff aus einem Melamin/Formaldehyd-Kondensationsprodukt mit einem Molverhältnis Melamin/Formadehyd im Bereich von 1 : 1 und 1:5 hergestellt wurde.

6. Verfahren nach einem der Ansprüche 1 bis 5, **dadurch gekennzeichnet, dass** der offenzellige Schaumstoff mit einem Hydrophobierungsmittel ausgerüstet ist.

7. Verfahren nach einem der Ansprüche 1 bis 6, **dadurch gekennzeichnet, dass** als Arbeitsgefäß ein Kulturröhrchen, Flasche oder Kolben für medizinische oder mikrobiologische Arbeiten verwendet wird.

8. Verfahren nach einem der Ansprüche 1 bis 7, **dadurch gekennzeichnet, dass** die Behandlung im Autoklaven unter Wasserdampf bei 120 bis 140°C und Druck im Bereich von 1 bis 4 bar durchgeführt wird.

9. Verfahren nach einem der Ansprüche 1 bis 6, **dadurch gekennzeichnet, dass** die Behandlung trocken und bei einer Temperatur im Bereich von 140 bis 220°C durchgeführt wird.

10. Verfahren nach einem der Ansprüche 1 bis 6, **dadurch gekennzeichnet, dass** die Behandlung unter Verwendung von Mikrowellenenergie durchgeführt wird

## Claims

1. A method of sterilizing working vessels, wherein the opening of the working vessel is closed by means of an open-celled foam based on an aminoplastic and treated at temperatures above 100°C.

2. A method of decontaminating material contaminated with microorganisms by treatment at temperatures above 100°C, wherein the material is introduced into a working vessel before the treatment and the vessel is closed by means of an open-celled foam based on an aminoplastic.

3. The method according to claim 1 or 2, wherein the opening of the working vessel is closed by means of an open-celled foam based on a melamine-formaldehyde condensation product.

4. The method according to any of claims 1 to 3, wherein the open-celled foam has a specific density in the range from 5 to 100 g/l.

5. The method according to any of claims 1 to 4, wherein the open-celled foam has been produced from a melamine-formaldehyde condensation product having a molar ratio of melamine to formaldehyde in the range from 1:1 to 1:5.

6. The method according to any of claims 1 to 5, wherein the open-celled foam has been treated with a hydrophobicizing agent.

7. The method according to any of claims 1 to 6, wherein a culture tube, bottle or flask for medical or microbiological work is used as working vessel.

8. The method according to any of claims 1 to 7, wherein the treatment is carried out under steam at from 120 to 140°C and a pressure in the range from 1 to 4 bar in an autoclave.

9. The method according to any of claims 1 to 6, wherein the treatment is carried out dry at a temperature in the range from 140 to 220°C.

10. The method according to any of claims 1 to 6, wherein the treatment is carried out using microwave energy.

## Revendications

1. Procédé de stérilisation de récipients de travail, **caractérisé en ce que** l'ouverture du récipient de travail est scellée avec une mousse à cellules ouvertes à base d'un aminoplaste et traitée à des températures supérieures à 100 °C.

2. Procédé de décontamination d'un matériau contaminé avec des microorganismes par traitement à des températures supérieures à 100 °C, **caractérisé en ce que** le matériau est introduit dans un récipient de travail avant le traitement et scellé avec une mousse à cellules ouvertes à base d'un aminoplaste.

3. Procédé selon la revendication 1 ou 2, **caractérisé en ce que** l'ouverture du récipient de travail est scellée avec une mousse à cellules ouvertes à base d'un produit de condensation mélamine/formaldéhyde.

4. Procédé selon l'une quelconque des revendications 1 à 3, **caractérisé en ce que** la mousse à cellules ouvertes présente une densité spécifique dans la plage allant de 5 à 100 g/l.

5. Procédé selon l'une quelconque des revendications 1 à 4, **caractérisé en ce que** la mousse à cellules ouvertes a été fabriquée à partir d'un produit de condensation mélamine/formaldéhyde ayant un rapport
molaire mélamine/formaldéhyde dans la plage allant de 1:1 à 1:5.

6. Procédé selon l'une quelconque des revendications 1 à 5, **caractérisé en ce que** la mousse à cellules ouvertes est munie d'un agent d'hydrophobation.

7. Procédé selon l'une quelconque des revendications 1 à 6, **caractérisé en ce qu'**un tube de culture, une bouteille ou un flacon pour travaux médicaux ou microbiologiques est utilisé en tant que récipient de travail.

8. Procédé selon l'une quelconque des revendications 1 à 7, **caractérisé en ce que** le traitement est réalisé dans des autoclaves sous vapeur d'eau de 120 à 140 °C et à une pression dans la plage allant de 1 à 4 bar.

9. Procédé selon l'une quelconque des revendications 1 à 6, **caractérisé en ce que** le traitement est réalisé à sec et à une température dans la plage allant de 140 à 220 °C.

10. Procédé selon l'une quelconque des revendications 1 à 6, **caractérisé en ce que** le traitement est réalisé en utilisant une énergie micro-onde.
